# EUROPEAN PATENT APPLICATION

(11) **EP 3 900 683 A1**
(43) Date of publication of application: **27.10.2021**
(21) Application number: 21167101.1
(22) Date of filing: 07.04.2021
(51) Int. Cl.: A61F 9/00

(54) **CANNULA AND CONTAINER OF OPHTHALMIC SOLUTIONS**

(30) Priority: 24.04.2020 IT 202000008863
(71) Applicant: Spinnato, Antonello, 22020 San Fermo della Battaglia (CO) (IT)
(72) Inventor: Spinnato, Antonello, 22020 San Fermo della Battaglia (CO) (IT)
(74) Representative: Biesse S.r.l.

(57) **Abstract**

A disposable cannula for administering liquid ophthalmic solutions in the eye of a user is described. The cannula comprises a body extending along a longitudinal direction between a first end, that can be constrained to a container containing an ophthalmic solution, and a second end, at which at least one delivery hole for delivering the ophthalmic solution is provided. The second end forms an angle with respect to the body of the cannula, i.e. deviates with respect to the body of the cannula, and the at least one delivery hole is on the side surface of the second end of the cannula. Advantageously, the second end comprises a closed distal section, a side surface through which one or more delivery holes for delivering the ophthalmic solution are present, and a proximal section for connecting with the rest of the body of the cannula. In practice, the second end of the cannula operates as a small foot from which the ophthalmic solution is delivered along a direction not parallel to the body of the cannula. A second aspect concerns a set comprising the cannula and a single-dose or multi-dose container containing an ophthalmic solution. The container is provided with an opening through which the ophthalmic solution can be delivered into the cannula. The set is made in one piece or in two pieces.

## Description

### Field of the invention

The present invention concerns a disposable cannula and a set comprising the cannula and a container for delivering ophthalmic solutions.

### State of the art

In the context of the present invention, the expression *ophthalmic solution* denotes a liquid preparation intended to be released onto the surface of the eye in the form of drops. The ophthalmic solutions can have different purposes: they can, for example, be used to substitute or supplement normal tears, or they can be medicines, such as antibiotics, anti-inflammatories, antihistamines, ophthalmic gels, etc. for the treatment of diseases or allergies.

Decongestant eye drops, preparations aimed at supplementing deficient tear secretion and at restoring the physiological conditions of the tear film, and the antihistamines antagonists of allergies, such as for example seasonal allergies to pollens, are among the most common ophthalmic solutions.

Currently, the ophthalmic solutions are marketed in containers which can conceptually be classified into two categories: multi-dose containers and mono-dose containers (disposable).

The present invention concerns both applications, i.e. concerns a cannula which can be used with a mono-dose container of ophthalmic solutions, i.e. a container containing a quantity of ophthalmic solution sufficient for a single application, or with a multi-dose container of ophthalmic solutions, i.e. a container containing a quantity of ophthalmic solution sufficient for several applications.

The containers of ophthalmic solutions are generally made of a yielding plastic material, i.e. are deformable. The ophthalmic solution is preserved sterile inside the container (antimicrobial agents, which ensure sterility for a predetermined amount of time, are added to the ophthalmic solution in the case of multi-dose containers). The container is provided with an outlet, which stays closed with a cap; when using it, the user provides to separate the cap from the container, thus uncovering a corresponding outlet hole, and to squeeze the container to reduce its volume. The pressure exerted with two fingers deforms the container, causing it to reduce its initial volume and the pouring out of the ophthalmic solution outside of the container through the outlet hole.

The cap is separable at a corresponding section of the container, for example at a weakened section or spout. The separation of the spout corresponds to breaking the material with which the container is made.

Generally, such deformable containers are also mono-dose, thus a single delivery corresponds to emptying the container. Instead, in other cases, the containers are multi-dose and the cap can be used to close the outlet hole again so that to preserve the ophthalmic solution that remained inside the container and to allow new deliveries.

Often, the containers of ophthalmic solutions are also named *droppers,* because it is possible to control the delivery of drops of the ophthalmic solution, i.e. to be able to deliver one drop at a time, by adequately adjusting the pressure exerted with the fingers onto the container within a time lapse.

The main drawback of containers of ophthalmic solutions lies in the fact that the outlet hole, which is created when the cap is separated from the container, often does not have a regular edge but, on the contrary, has irregularities which can scratch the eye of a user if put into contact with the ocular surface at the cornea and conjunctiva. The reason is simple: the cap cannot be severed with a blade, so that to avoid contaminating the ophthalmic solution and to preserve its sterility, but is literally teared off by the user. This circumstance sometimes induces diffidence in the user, who prefers neither to lean nor to even put the container near the eye during the delivery, with the consequence of making it difficult to properly direct the drops of ophthalmic solution into the eye.

A further drawback of the containers of ophthalmic solutions currently available lies in the fact that, aside from the consideration just made, they are not simple to use for people who have trouble keeping their eyelids open and the head slightly titled while administering the ophthalmic solution. In fact, some people subconsciously tend to close their eyelids (wink) when the ophthalmic solution is about to be released from a container placed in front of the eye. This particularly happens to elderly people who suffer from chronic pathologies, such as glaucoma, and who are forced to make daily applications of an ophthalmic solution containing beta blockers without anyone assisting them. It often occurs that the drops of ophthalmic solution end up on these people's eyelid or face, but not in their eye.

US 2016/0270956 describes a cannula for administering a medicinal liquid into the eye of a patient after a surgical procedure. The cannula has the characteristics contained in the preamble of claim 1. Moreover, this document describes that the end of the cannula from which the medicinal liquid is delivered is ring-shaped and the delivery hole for delivering the medicinal liquid opens at the central opening of the ring.

US 2007/0055208 also describes a cannula according to the preamble of claim 1. In this solution, the delivery hole coincides with the section itself of the second end from which the ophthalmic solution is delivered.

### Summary of the invention

Object of the present invention is to provide a solution which allows to overcome the drawbacks just described, in a simple way and for everyone.

A first aspect of the present invention thus concerns a disposable cannula according to claim 1 for administering liquid ophthalmic solutions on the outer surface of the eye of a user, i.e. on the outer surface of the cornea or conjunctiva.

In particular, the disposable cannula according to the invention comprises a body extending along a longitudinal direction between a first end, that can be constrained to a container containing an ophthalmic solution, and a second end, at which at least one delivery hole for delivering the ophthalmic solution is provided. In other words, the second end is provided with one or more delivery holes depending on the density of the ophthalmic solution used.

The container containing the ophthalmic solution, to which the cannula can be constrained, can be a container (disposable) of ophthalmic solutions, i.e. a container containing a quantity of ophthalmic solution sufficient for a single application, or a multi-dose container of ophthalmic solutions, i.e. a container containing a quantity of ophthalmic solution sufficient for several applications. The second end forms an angle with respect to the body of the cannula, i.e. is angled with respect to the body of the cannula, that is to say deviates with respect to the body of the cannula and does not extend along the longitudinal direction, and the at least one delivery hole is on the side surface of the second end of the cannula.

In practice the second end forms, with respect to the cannula, an angle different from zero or different from a flat 180° angle, for example within an interval of 1° and 179°.

Advantageously, the second end of the cannula comprises a distal section, i.e. the end section, which is closed i.e. sealed with respect to the outside, a side surface through which one or more delivery holes for delivering the ophthalmic solution are present, and a proximal section for connecting with the rest of the body of the cannula.

The solution just described allows to make sure the delivery of the ophthalmic solution does not occur longitudinally with respect to the cannula, but by an angle, i.e. transversely with respect to the cannula. In practice, the second end of the cannula operates as a small foot from which the ophthalmic solution is delivered, at the side surface, orthogonally to the small foot and thus along a direction not parallel to the body of the cannula.

Being able to deliver the ophthalmic solution angled with respect to the cannula is advantageous because the container can be held out of sight of the user administering the drops. The user can hold the container away from the eye and only bring the second end of the cannula close to the surface of the eye by holding it almost parallel to the face. The user will thus not think that he could accidentally injure himself by inserting the cannula into the eye. The second end of the cannula will anyhow not be facing the surface of the eye, but will point at the opposite part: the drops will anyhow come out of the delivery hole or holes and will fall onto the surface of the eye, even if the tip of the second end will be facing the nose.

Moreover, also whenever the second end of the cannula accidentally comes into contact with the surface of the eye, it will not cause injuries or scratches since the second end operates as a small foot and will lean against the surface of the eye with its side surface and not with its end section. In other words, the side surface of the second end of the cannula will not be able to injure the eye as would a pointy element, for example, as would the cannula if the second end was straight and did not form an angle with the body of the cannula.

Thus, ultimately, the particular shape of the cannula facilitates the administration of the ophthalmic solution also for subjects who subconsciously are afraid of injuring themselves and tend to close their eyelids when nearing the container. Moreover, the shape of the cannula, with the second end angled with respect to the body of the cannula, prevents the user from injuring himself whenever the cannula is accidentally brought into contact with the ocular surface.

The cannula described above facilitates and improves the administration of the ophthalmic solution, also because it allows to carry out the application of the ophthalmic solution directly at the level of the conjunctival surface, without having to tilt the head, but by keeping the head vertical and by lowering the eyelid with a hand. This ensures that the drops of ophthalmic solution are delivered directly at the absorption site, which is in fact the conjunctiva.

In practice, the second end of the cannula opens outward only at the at least one delivery hole, i.e. has a closed end section through which the ophthalmic solution cannot pass.

Preferably, the end section has a smooth surface and a rounded profile, so that to be atraumatic in the event of contact with the eye.

Preferably, the cannula has two or more delivery holes positioned close to one another on the side surface of the second end of the cannula. This characteristic allows to direct the ophthalmic solution fan-like and to distribute it on a wide surface.

In the preferred embodiment, the second end of the cannula forms an angle with the body of the cannula of between 30° and 60°, and preferably forms an angle of 45°. This configuration allows, for example, to lay a side of the hand on a cheekbone, such as the right cheekbone, and to bring the second end of the cannula at the left eye, by keeping the hand leaned and limiting tremors.

The cannula can be made in four versions:
- a version wherein the first end can be sealingly constrained to a container of an ophthalmic solution,
- a second version wherein the first end can be sealingly inserted into a fitting of the container or constrained to the container,
- a third version wherein the first end is itself shaped like a fitting that can be constrained to the container, or else
- a fourth version wherein the cannula is made in one piece with the container.

For example, the first end can be shaped like a fitting of the Luer type and can thus also be sealingly constrained to a syringe used as a container of the ophthalmic solution.

Preferably, the delivery hole or holes open at the side surface of the second end, from the opposite part with respect to the first end, i.e. from the opposite part with respect to the container of the ophthalmic solution.

Preferably, the cannula is made of a plastic material, for example of a sterilizable polymer, or steel, and is also packaged individually in sterile blisters. Preferably, the cannula is made of polyethylene, polypropylene or ethylene-vinyl acetate (Eva).

The cannula can be made of a flexible polymeric material.

Preferably, the length of the second end of the cannula is equal to a tenth of the length of the body of the cannula.

In the preferred embodiment, the cannula has an almost cylindrical or conical shape, so that the outer surface is rounded and cannot cause damages following an accidental contact with the eye.

Preferably, the outer diameter of the body of the cannula is equal to 0.8 mm, the length of the body of the cannula is of 35 mm, excluding the second end, and the length of the second end is of 3.5 mm.

A second aspect of the present invention concerns a set according to claim 10 comprising the cannula described above and a mono-dose or multi-dose container.

In particular, the container contains an ophthalmic solution and is provided with an opening through which the ophthalmic solution can be delivered into the cannula.

The set can be made in one piece or in two or more pieces according to one of the following methods:
- the container comprises a cap for closing the opening, the cap is removable, and the container can be sealingly constrained to the cannula or to a fitting of the cannula, at the opening, or
- the container is made in one piece with the cannula.

In the first variant, the cannula and the container can be sold separately, for example each in a blister.

In this first variant, the set can be made with a fitting to interpose between the cannula and the container, i.e. a fitting having a first seat, into which the first end of the cannula can be sealingly inserted, and a second seat, that can be sealingly fitted onto the opening of the container.

This is the case, for example, of a set comprising a cannula and a fitting constrained under pressure to a mono-dose container, or which are constrained under pressure or screwed to a multi-dose container.

In the second variant, the cannula is integral with the container, i.e. is in one piece with a container, for example disposable, molded and pre-filled.

Generally, the fitting can thus be integral with the cannula, i.e. the first end of the cannula can be shaped like a fitting, or the fitting can be made separately and can be sold together with the cannula or together with the container, or even separately.

Preferably, the container is deformable with the pressure of the fingers so that to cause the delivery of the ophthalmic solution.

Also, the container is preferably made of a sterilizable plastic material.

In the preferred embodiment, the container comprises an outer portion coated with an absorbing material, preferably cellulose. This characteristic allows the user to absorb any excess of ophthalmic solution from an angle of the eye.

In the second variant, the container can be made with the opening shaped like a fitting of the Luer type.

### Brief list of the figures

Further characteristics and advantages of the invention will be better highlighted by the review of the following detailed description of preferred, although not exclusive, embodiments depicted by way of example and without limitations, with the aid of the accompanying drawings, in which:
- figure 1 is a schematic exploded view of a first embodiment of a cannula and a set according to the present invention;
- figure 2 is a schematic view of a second embodiment of a cannula according to the present invention and a fitting;
- figure 3 is a sectional view, considered with respect to the sectional plane C-C, of the cannula and fitting shown in figure 2;
- figure 4 is a schematic view of a third embodiment of a cannula according to the present invention with a fitting;
- figure 5 is a sectional view, considered with respect to the sectional plane A-A, of the cannula and fitting shown in figure 4;
- figure 6 is a schematic view of the fitting shown in figures 2-5.

### Detailed description of the invention

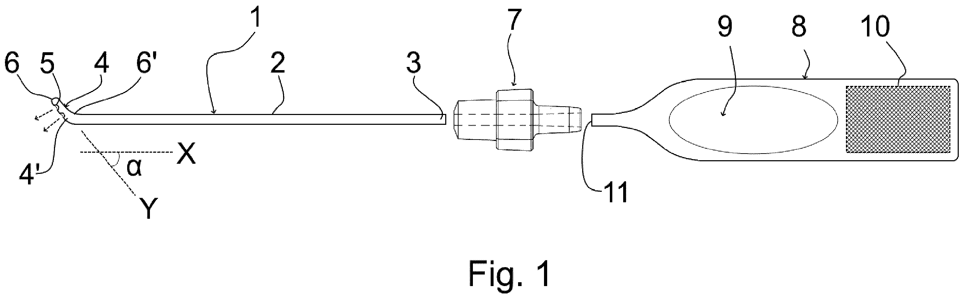

Figure 1 shows, with reference 1, a first embodiment of a disposable cannula according to the present invention, provided with a body 2 developing along a longitudinal direction parallel to the axis X. The cannula 1 develops itself between a first end 3, which is on the right part of the cannula 1 in figure 1, and a second end 4, which is on the left part of the cannula 1 in figure 1.

The cannula 1 is hollow so that to allow the passage of an ophthalmic solution 9. In fact, the first end 3 is open and can be inserted into a container 8 of the ophthalmic solution 9, or can be inserted into a fitting 7 for connecting with the container 8.

The second end 4 of the cannula 1 is not aligned with the rest of the body 2 along the longitudinal direction, but forms an angle α of between 30° and 60°, and preferably equal to 45°, with the rest of the body 2. In other words, the second end 5 is a branching of the body 2 extending along an axis Y incident with respect to the axis X, according to the angle α just described.

The end section 6 of the second end 4, i.e. the tip of the cannula 2, is closed: the delivery of the ophthalmic solution 9 cannot occur through the tip 6. The second end 4 extends between the end section 6 and a section 6' for connecting with the rest of the body 2 of the cannula 1, and is provided with a side surface 4', which is conical in the example shown in figure 1, through which at least one delivery hole 5 is made, and preferably several delivery holes 5 are made.

The delivery of the ophthalmic solution in fact occurs through the delivery holes 5 present laterally on the second end 4 of the cannula 1. Since, as described above, the second end 4 extends along the axis Y, the delivery of the ophthalmic solution from the delivery holes 5 occurs substantially along a direction orthogonal to the axis Y. This detail allows to administer the ophthalmic solution 9 with an angle with respect to the longitudinal extension direction of the cannula 1, thus achieving the advantages described in the summary of the invention.

The dotted arrows denote the exit direction of the ophthalmic solution from the delivery holes 5.

In the example shown in figure 1, the cannula 1 is made of a sterilizable plastic material, for example in an autoclave whenever the cannula is intended to be sold individually; the outer diameter of the body 2 is equal to 0.8 mm and the length of the body in the longitudinal direction, between the first end 3 and the portion 6' for connecting the second end 4, is equal to 35 mm. Clearly, this is an example: the dimensions described can differ depending on the need, for example for hospital, domestic use, etc.

In alternative to the polymeric material, the cannula 1 is also made of steel and, in this circumstance, is rigid.

Preferably, as shown in figure 1, the length of the second end 4 is equal to about a tenth of the length of the body 2 of the cannula 1, which in the example of figure 1 corresponds to 3.5 mm.

The diameter of the delivery holes 5 is preferably within the 0.1-0.3 mm interval and depends on the density of the ophthalmic solution which must be delivered.

Preferably, the delivery holes 5 are close to one another on the side surface 4' of the second end 4 of the cannula 1 so that to create a curtain of ophthalmic solution during the administration onto the outer surface of the eye of the user.

The first end 3 is made with different shapes depending on whether it is provided to be joined directly with the container 8 or with a fitting 7 which can be inserted between the cannula 1 and the container 8. For example, in an embodiment (not shown), the first end 3 is itself shaped like a Luer type fitting.

The cannula 1 and the container 8 shown in figure 1 form a set according to the present invention. In particular, the cannula 1 and the container 8 can be sold separately, for example in specific blisters, or together as a modular kit. In an embodiment, the cannula 1 and the container 8 are made in one piece, without interruptions.

The container 8 is preferably made of a yielding and sterilizable plastic material, so that to allow the user to squeeze and deform the container itself and to cause the coming out of the ophthalmic solution. The container 8 comprises an opening 11 intended to be put in fluidic communication with the cannula 1 or a fitting 7, whenever these elements are made separately, or the opening 11 merges into the cannula 1 whenever the set is made in one piece, i.e. with the cannula 1 and the container 8 joined at the origin.

Whenever needed, the opening 11 can be made like a Luer fitting so that to allow the connection, under pressure, with the cannula 1.

In the solution made in one piece, the first end 3 of the cannula 1 is joined at the opening 11 of the container 8.

Preferably, the container 8 comprises an outer portion coated with an absorbing material 10, for example cellulose, whose function is to allow the user to absorb any excess of ophthalmic solution from an angle of the eye.

The set just described can further comprise a fitting 7, for example with connections of the Luer type. The fitting 7 can be made as an element distinct from the cannula 1 and from the container 8, and can be interposed between them as a connecting element, or can be made in one piece with the cannula 1 or the container 8.

The operation of the set is simple: once the set has been properly assembled, the user brings the second end 4 of the cannula 1 close to the eye and provides to press the container 8 so that to deform it and cause the coming out of the ophthalmic solution through the opening 11, the fitting 7 - if present -, the cannula 1 and through the holes 5.

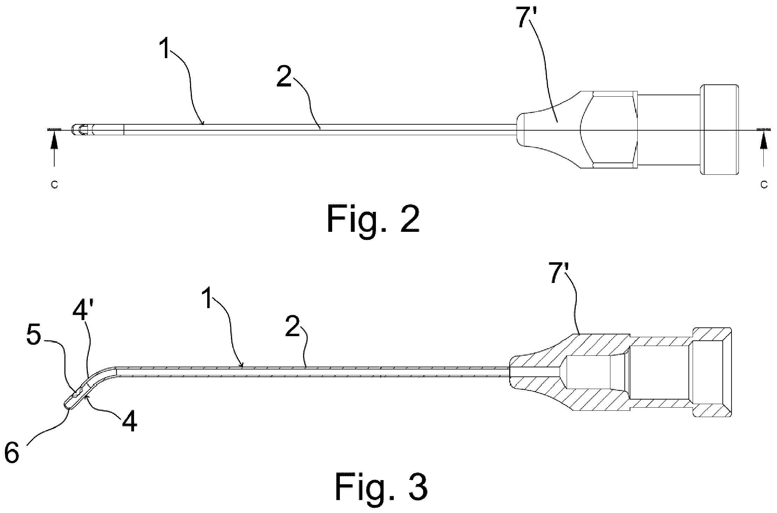

A second embodiment of a cannula 1 according to the present invention is shown in figures 2 and 3 with a fitting 7' thermo-sealed to the cannula 1. The container of the ophthalmic solution is not shown in these figures.

As visible in figure 3, which is a section considered along the sectional plane C-C of the cannula shown in figure 2, this embodiment is different from the preceding one in that the cannula 1 is provided with a single delivery hole 5 obtained in the side surface 4' of the second end 4. At the side surface 4', the delivery hole 5 preferably has a length of 1 mm and opens at the inner cavity of the cannula 1 at 1.5 mm from the distal section 6.

In this embodiment, the outer diameter of the body 2 is of 0.8 mm, whereas the inner diameter of the body 2 is of 0.5 mm.

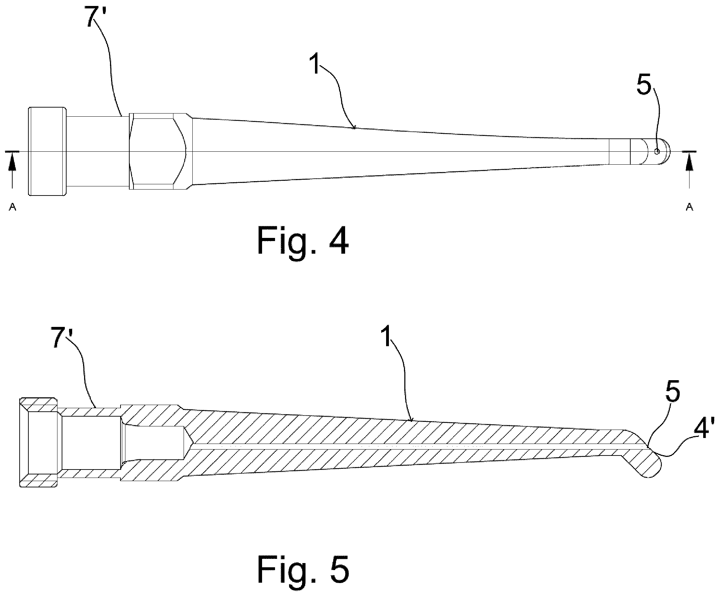

A third embodiment of a cannula 1 made in one piece with a fitting 7' is shown in figures 4 and 5.

Also in this embodiment, the cannula 1 is provided with a single delivery hole 5 at the side surface 4'.

Unlike the two preceding embodiments, the delivery hole 5 is coaxial with respect to the inner cavity of the cannula extending on an axis parallel and not coincident with the symmetry axis of the fitting 7'.

Generally, the cannulas 1 described above can be combined with a container of ophthalmic solutions both of the mono-dose and multi-dose type and can be made of a material such as polyethylene, polypropylene or ethylene-vinyl acetate (Eva).

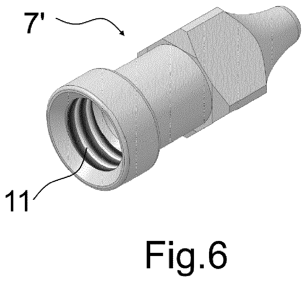

The fitting 7' depicted in figures 2-5 is schematically shown in figure 6. The fitting 7' is arranged to be assembled, at a first end, with a cannula 1, and at a second end, opposite the first, with a container 8 by means of a luer slip pressure system or by means of a thread 11, at which a container 8 provided with a screw cap can be screwed.

## Claims

1. A disposable cannula (1) for administering liquid ophthalmic solutions on the outer surface of the eye of a user, comprising a body (2) extending along a longitudinal direction (X) between a first end (3), that can be constrained to a container (8) of an ophthalmic solution (9), and a second end (4), at which at least one delivery hole (5) for delivering the ophthalmic solution (9) is provided, wherein the second end (5) forms an angle (α) different from zero with respect to the body (2) of the cannula (1) and wherein the at least one delivery hole (5) is arranged on the side surface (4') of the second end (4) of the cannula (1), **characterized in that** the second end (4) comprises a closed distal section (6), a side surface (4') through which one or more delivery holes (5) for delivering the ophthalmic solution (9) are present, and a proximal section (6') for connecting with the rest of the body of the cannula.

2. Cannula (1) according to claim 1, wherein the second end (4) opens outward only at the at least one delivery hole (5).

3. Cannula (1) according to claim 1 or claim 2, comprising two or more delivery holes (5) positioned close to one another on the side surface (4') of the second end (4) of the cannula (1).

4. Cannula (1) according to any one of preceding claims 1-3, wherein the angle (α) between the second end (4) of the cannula (1) and the body (2) is between 30° and 60°, and is preferably equal to 45°.

5. Cannula (1) according to any one of preceding claims 1-4, wherein the first end (3) can be sealingly constrained to a container (8) of an ophthalmic solution, or can be sealingly inserted into a fitting (7, 7') that can be constrained to such container (8), or is itself shaped like a fitting that can be sealingly constrained to such container (8).

6. Cannula (1) according to any one of preceding claims 1-5, wherein the at least one delivery hole (5) opens at the side surface (4') of the second end (4), from the opposite part with respect to the first end (3).

7. Cannula (1) according to any one of preceding claims 1-6, made of a sterilizable plastic material or steel.

8. Cannula (1) according to any one of preceding claims 1-7, wherein the length of the second end (4) is equal to a tenth of the length of the body (2).

9. Cannula (1) according to any one of preceding claims 1-8, wherein the outer diameter of the body (2) is equal to 0.8 mm, the length of the body is of 35 mm and the length of the second end (4) is of 3.5 mm.

10. A set comprising the cannula (1) according to any one of preceding claims 1-9 and a container (8) provided with an opening (11) and containing an ophthalmic solution (9), wherein:
- the container (8) comprises a cap for closing the opening (11), the cap is removable, and the container (8) can be sealingly constrained to the cannula (1) or to a fitting (7, 7') of the cannula (1), at the opening (11), or
- the container (8) is made in one piece with the cannula (1).

11. Set according to claim 10, wherein the container (8) is deformable with the pressure of the fingers.

12. Set according to claim 10 or claim 11, wherein the container (8) is made of a sterilizable plastic material.

13. Set according to any one of preceding claims 10-12, wherein the container (8) comprises an outer portion coated with an absorbing material (10) for the ophthalmic solution, said absorbing material (10) preferably being made of cellulose.

14. Set according to any one of preceding claims 10-13, comprising a fitting (7, 7') having a first seat, into which the first end (3) of the cannula (1) can be sealingly inserted, and a second seat, that can be sealingly fitted onto the opening (11) of the container.

15. Use of the set according to any one of preceding claims 10-14 for delivering an ophthalmic solution (9) initially contained inside the container (8), on the outer surface of the eye of a user through the at least one delivery hole (5) present on the second end (4) of the cannula (1), according to an angle (α) different from zero with respect to the longitudinal extension direction (X) of the body (2) of the cannula (1).
